Europäisches Patentamt
European Patent Office
Office européen des brevets

(19)

(11) Publication number: 0 006 987
A1

# EUROPEAN PATENT APPLICATION

(12)

(21) Application number: 79101596.9

(22) Date of filing: 23.05.79

(51) Int. Cl.[3]: C 07 D 239/48
A 61 K 31/505

(30) Priority: 24.05.78 GB 2193878

(43) Date of publication of application:
23.01.80 Bulletin 80/2

(84) Designated Contracting States:
BE CH DE FR GB LU NL SE

(71) Applicant: THE WELLCOME FOUNDATION LIMITED
183-193 Euston Road
London NW1 2BP(GB)

(72) Inventor: Bushby, Stanley Robert Morris
3744 St. Marks Road
Durham North Carolina 27707(US)

(72) Inventor: Burchall, James Joseph
1325 Kershaw Drive
Raleigh North Carolina 27609(US)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

(54) **2,4-Diamino-5-benzylpyrimidines, especially for the treatment of microbial infections, pharmaceutical compositions containing these compounds and processes for preparing these compounds.**

(57) Pharmaceutical compositions containing 2,4-diamino-5-(3, 5-dialkyl-4-hydroxybenzyl) pyrimidines wherein the alkyl groups contain from 2 to 4 carbon atoms are useful in the treatment of bacterial infections. The first medical use of such compounds, novel chemical compounds wherein the alkyl groups are propyl or butyl, except the di-i-propyl compound, a process for preparing the novel compounds and chemical intermediates used in their preparation are also disclosed.

EP 0 006 987 A1

Croydon Printing Company Ltd.

0006987

TITLE MODIFIED
see front page



B 293

"Benzylpyrimidine Compositions"

The present invention relates to 2,4-diamino-5-benzylpyrimidines, to pharmaceutical compositions containing them, to processes for preparing them and their compositions and to their use in the treatment of bacterial infections.

Certain 2,4-diamino-5-benzylpyrimidines have been demonstrated to exhibit useful pharmaceutical properties and to be sulphonamide potentiators.

Thus, U.K. Patent Specification No.875,562 discloses *inter alia* compounds of the formula (I):

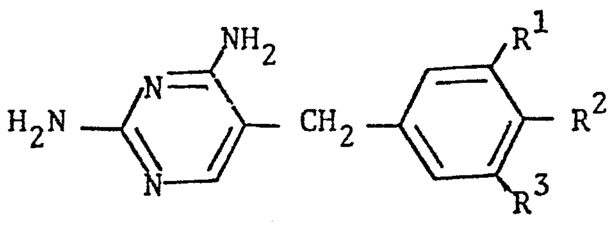

(I)

wherein $R^1$, $R^2$ and $R^3$ are the same or different $C_{1-4}$ alkoxy groups. Trimethoprim, 2,4-diamino-5-(3,4,5-trimethoxybenzyl)pyrimidine, is specifically disclosed in U.K. Patent No.875,562 and is the most active general antibacterial agent amongst the 2,4-diamino-5-benzylpyrimidines known to date. Trimethoprim has been used extensively over the last decade in human therapy in combination with various sulphonamides, and in particular with sulphamethozazole, for the treatment of bacterial infections, and has been recommended for the treatment of Neisseria gonorrhaea (Rodin and Seth, *Brit*. *J*. *Vener*. *Dis*. 1972, *48*, 517).

2,4-Diamino-5-benzylpyrimidines having a hydroxy group in the 4-position of the phenyl ring and possessing antibacterial activity are disclosed in U.K. Patent No. 1,128,234 and Belgian Patent No.846,397.

10). A process for preparing a compound of the formula (VII) which comprises:

(i) the reaction of a compound of the formula (VIII):

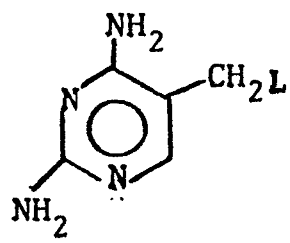

(VIII)

wherein L is a leaving group, with the appropriately substituted phenol of the formula (IX):

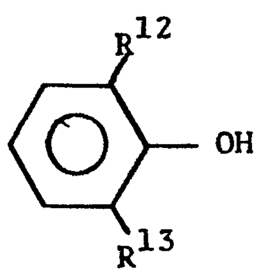

(IX)

wherein $R^{12}$ and $R^{13}$ are as hereinbefore defined,

(i) the reaction of a compound of the formula (X):

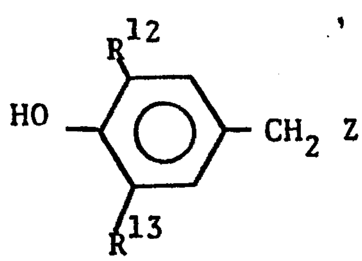

(X)

wherein Z is a hydroxy or disubstituted amino group or a halogen atom and $R^{12}$ and $R^{13}$ are as hereinbefore

defined with a compound of the formula (XI):

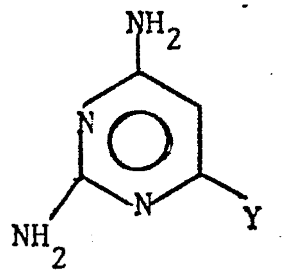

(XI)

wherein Y is a hydrogen or halogen atom or an alkylthio, aralkylthio or mercapto group, and then, in the case where Y is an alkylthio or aralkylthio group, removing this group by hydrogenolysis,

(iii) cleaving a group $R^{14}$, wherein $R^{14}$ is a protecting group as hereinbefore defined, from a compound of the formula (XII):

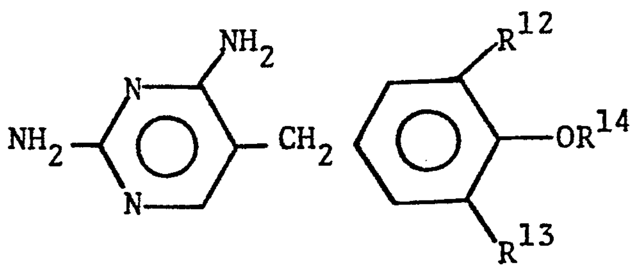

(XII)

wherein $R^{12}$, $R^{13}$ are as hereinbefore defined,

(iv) the reaction of a compound of the formula (XIII):

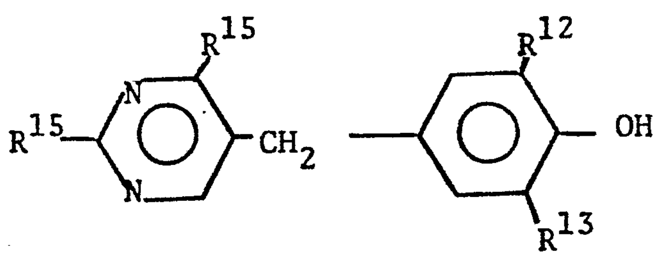

(XIII)

intermediates in the preparation of pharmacologically active compounds they were not themselves disclosed as having any pharmacological activity.

It has now been found that a group of compounds within the formula (V) wherein $R^7$ and $R^9$ are the same or different $C_{2-4}$ alkyl groups are extremely active *in vitro* and *in vivo* against the bacteria *Neisseria gonorrhoeae* and Neisseria *meningitidis*. Many of the compounds within this group are more than twice as active *in vivo* as trimethoprim against these *Nesseria* organisms and in some cases they are as much as 15 times more active than trimethoprim *in vitro*. This discovery has extremely important implications in the field of hyman therapy in view of the recent discovery (New Scientis, 28th April 1977)pp. 202, 205 of penicillin resistant strains of *Neisseria*. Penicillins have previously been widely used in the treatment of *Neisseria*.

These compounds are also useful and effective in the treatment of infections in mammals caused by anaerobic microorganisms such as species of *Bacteroides*, *Fusobacterium*, *Peptococcus*, *Peptostreptococcus*, and *Clostridium*, for example *Bacteroides fragilis*, *B. thetaiotaomicron*, *B. vulgatus*, *Clostridium*

*perfringens*, *C. tetani*, *C. sphenoides*, and *Fusobacterium necrophorum*. Pathological conditions caused by these microorganisms include sepis of deep wounds such as those due to trauma, e.g. deep puncture wounds, and post operative infection.

Accordingly, the present invention provides pharmaceutical compositions which comprises a compound of the formula (VI):

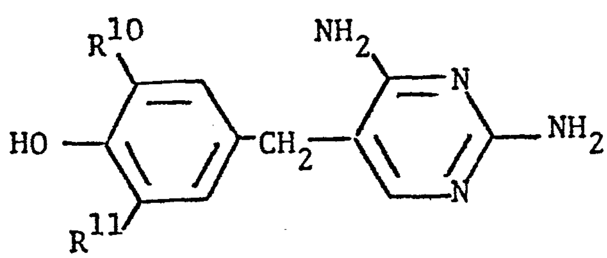

(VI)

wherein $R^{10}$ and $R^{11}$, which may be the same or different, are $C_{2-4}$ alkyl groups, or pharmaceutically acceptable salt thereof, together with a pharmaceutical acceptable carrier.

The alkyl groups $R^{10}$ and $R^{11}$ may be either straight or branched chain alkyl groups. $R^{10}$ and $R^{11}$ may therefore be ethyl, n-propyl, i-propyl, n-butyl, s-butyl, i-butyl or t-butyl.

It has been found that those compounds of the formula (VI) wherein $R^{10}$ $R^{11}$ are branched- chain $C_{2-4}$ alkyl groups are less readily metabolised in vivo than those compounds wherein $R^{10}$ and $R^{11}$ are straight-chain alkyl groups. Accordingly, $R^{10}$ and $R^{11}$ are preferably the same or different branched -chain $C_{2-4}$ alkyl groups, that is to say i-propyl, s-butyl, i-butyl or t-butyl. Preferably $R^{10}$ and $R^{11}$ are the same.

Therefore preferred compounds for inclusion within the pharmaceutical compositions of the present invention include:-

(i) 2,4-Diamino-5-(3,5-di-t-butyl-4-hydroxybenzyl)pyrimidine;

(ii) 2,4-Diamino-5-(3,5-di-s-butyl-4-hydroxybenzyl)pyrimidine;

(iii) 2,4-Diamino-5-(3,5-di-i-butyl-4-hydroxy-benzyl)pyrimidine;

(iv) 2,4-Diamino-(4-hydroxy-3,5-di-i-propyl-benzyl)pyrimidine; and pharmaceutically acceptable salts thereof.

The present invention also provides the compounds of the formula (VI),or pharmaceutically acceptable salts thereof, for use in medicine for the treatment of microbial injections.In one preferred aspect the microbial infections will be bacterial infections,

particularly those caused by Neisseria gonorrhoeae And Neisseria meningitidis. In a further preferred aspect the microbial infections will be anaerobic infections.

Certain compounds within the formula (VI) are novel and the present invention extends to these compounds. Accordingly, the present invention provides compounds of the formula (VII):

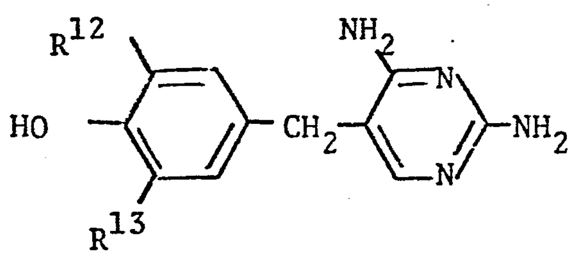

(VII)

wherein $R^{12}$ and $R^{13}$, which may be the same or different arepropyl or butyl, except that $R^{12}$ cannot be i-propyl when $R^{13}$ is i-propyl, and pharmaceutically acceptable salts thereof.

Preferred compounds of the formula (VII) are those wherein $R^{12}$ and $R^{13}$, which may be the same or different, are i-propyl, i-butyl s-butyl or t-butyl, except when $R^{12}$ and $R^{13}$ are both i-propyl, and pharmaceutically acceptable salts thereof.

Most suitably $R^{12}$ and $R^{13}$ are the same. Thus, preferred novel compounds of the present invention are:-

(i) 2,4-Diamino-5-(3,5-di-t-butyl-4-hydroxybenzyl)pyrimidine;

(ii) 2,4-Diamino-5-(3,5-di-s-butyl-4-hydroxybenzyl)pyrimidine;

(iii) 2,4-Diamino-5-(3,5-di-i-butyl-4-hydroxybenzyl)pyrimidine; and pharmaceutically acceptable salts thereof.

Suitable acid addition salts of the compounds of formula (VI) include those formed with both organic and inorganic acids. Such acid addition salts will normally be pharmaceutically acceptable. Thus, preferred salts include those formed from hydrochloric, sulphuric, citric, tartaric, phosphoric, lactic, pyruvic, acetic, succinic, fumaric, maleic and oxaloacetic acids. It will be readily apparent that the compounds of the formula (VI) have two free amino groups and are capable of forming both mono- and di-acid addition slats. Mono-acid addition salts are preferred. The compounds of the formula (VI) will be present in the compositions of the present invention in an effective unit dosage form, that is to say in an amount sufficient to be effective against the

bacterial organism in vivo.

The pharmaceutically acceptable carriers present in the compositions of the present invention are materials recommended for the purpose of administering the medicament. These may be liquid, solid or gaseous materials, which are otherwise inert or medically acceptable and are compatible with the active ingredients. For the purposes of this invention non-sterile water and dimethylsulphoxide are not considered to be pharmaceutically acceptable carriers.

These pharmaceutical compositions may be given pharmaceutically, orally, used as a suppository, applied as an opthalmic solution, or applied topically as an ointment, cream or powder. However, oral and parenteral administration of the compositions is preferred.

For oral administration, fine powders or granules can contain diluting, dispensing and/or surface active agents, and may be presented in a draught, in water or in a syrup, in capsules or sachets in the dry state or in a non-aqueous suspension wherein suspending agents may be included, or in a suspension in water or syrup. Where desirable or necessary,

flavouring, preserving, suspending, thickening or emulsifying agents can be included. When a suspension is prepared in water according to the present invention at least one of such agents will be present.

For parenteral administration, the compounds may be presented in sterile aqueous injection solution which may contain antioxidants or buffers.

As stated above, free base or a salt thereof may be administered in its pure form unassociated with other additives in which case a capsule or sachet is the preferred carrier.

Alternatively the active compound may be presented in a pure form as an effective unit dosage, for instance, compressed as a tablet or the like.

It may be advantageous to include the compounds of formula (VI) in a pharmaceutical composition which may include other active ingredients for example p-amino benzoic acid competitors such as sulphonamides.

Of the known p-amino benzoic acid competitors, the following sulphonamide compounds (or pharmaceutically acceptable salts thereof) are useful:- Sulfanilamide, Sulfadiazine, Sulfamethisazole, Sulfapyridine, Sulfathiazole, Sulfamerazine, Sulfamethazine, Sulfisoxazole, Sulformethoxine, 2-(p-Aminobenzene)sulfonamido-3-methoxypyrazine (Kelfizina), p,$p^1$-Diaminodiphenylsulfone, α-Amino-p-toluenesulfonamide, 5-Sulfanilamido-2,4-dimethyl pyrimidine, 4-($N^1$-Acetyl sulfanilamido)-5,6-dimethoxy pyrimidine, 3-Sulfanilamido-4,5-dimethyl isoxazole, 4-Sulfanilamido-5-methoxy-6-decyloxy pyrimidine, sulfamonomethoxine, 4-p-(8-Hydroxy-quinilinyl-4-azo)-phenyl sulfanilamido-5, 6-Dimethoxy pyrimidine, Sulfadimethoxine, Sulfadimidine, Sulfamoxole, Sulfadoxine, Sulfaguanidine, Sulfathiodimethoxine, Sulfamethoxazole, Sulfaquinoxaline, and p-(2-Methyl-8-hydroxy-quinolinyl-(5)-azo)-phenyl sulfanilamido-5,6-dimethoxy pyrimidine.

However, the most preferred combinations include those containing Sulfadiazine, Sulfamethoxazole, Sulfadoxine, Sulfaquinoxaline, Sulfadimidine, Sulfamazole or Sulphaguanidine.

Other compounds which may be included are, for example, medically inert ingredients, e.g., solid and liquid diluents such as lactose, glucose, starch or calcium phosphate for tablets or capsules; olive oil or ethyl oleate for soft capsules; and water or vegetable oil for suspensions or emulsions; lubricating agents such as talc or magnesium stearate; gelling agents such as colloidal clays, thickening agents such as gum tragacanth or sodium alginate: and other therapeutically acceptable accessory ingredients such as humectants, preservatives, buffers, and antioxidants which are useful as carriers in such formulations.

Tablets or other forms of presentation provided in discrete units may conveniently contain an amount of compound of the formula (VI) which is effective at such dosage or as a multiple of the same, for instance, units containing 2.5 mg. to 150 mg., usually around 30 to 100 mg.

In the treatment of infections caused by anaerobic microorganisms the compounds of formula (VI) and their pharmaceutically acceptable salts may be administered orally, parenterally or as a suppository in the appropriate pharmaceutical formulations and

and at the dose ranges described above. The compounds of formula (VI) and their pharmaceutically acceptable salts may be administered alone or in combination with sulfonamides as indicated above.

The pharmaceutical compositions of the present invention will be prepared by the admixture of a compound of the formula (VI) with a pharmaceutically acceptable carrier. Other active ingredients, such as a sulphonamide, or conventional pharmaceutical excipients may be admixed as required.

Still another aspect of the present invention provides a method of treating bacterial infections in mammals by the administration of an effective non-toxic antibacterial amount ofa compound of the formula (VI) or a pharmaceutically acceptable salt thereof, or a composition as hereinbefore described.

As indicated above, the compounds of the formula (VI) are generally useful in treating such infections by topical application or by oral administration or injection. The compounds of the formula (VI) are normally administered at a dose of from 1mg/kg to 30mg/kg per day. The dose range for adult humans is generally from 25 to 1000mg/day and preferably

50 to 300mg/day.

The compounds of the formula (VII) and their pharmaceutically acceptable salts may be prepared by several routes. Thus the compounds may be prepared by: (i) the method described in U.K. Patent No. 1,413,471 which comprises reacting a compound of the formula (VIII)

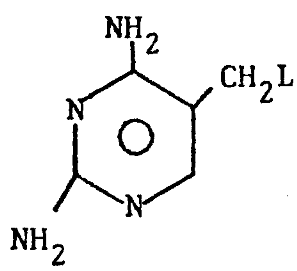

(VIII)

wherein L is a leaving group, with the appropriately substituted phenol of the formula (IX):

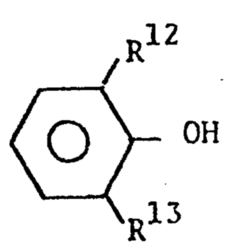

(IX)

wherein $R^{12}$ and $R^{13}$ are as hereinbefore defined.

Suitable leaving groups L include hydroxy groups, halogen atoms such as bromine and chlorine

and the anionic residue of a carboxylic or sulphonic acid for example methane sulphonic acid or toluene-p-sulphonic acid. Other suitable leaving groups not exemplified in U.K. Patent No. 1413471 include amino groups, preferably di-$C_{1-6}$ alkylamino groups. This reaction is normally carried out in the presence of a strong acid catalyst such as a strong mineral acid, e.g. hydrochloric acid, or a carboxylic or sulphonic acid; (ii) a method similar to that described in U.K. Patent Nos. 1,128,234 and 1,401,612 which comprises reacting a compound of the formula (X):

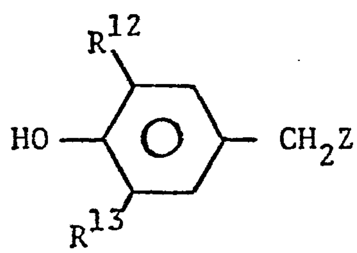

(X)

wherein Z is a hydroxy or di-substituted amino group or a halogen atom and $R^{12}$ and $R^{13}$ are as hereinbefore defined, with a compound of the formula (XI):

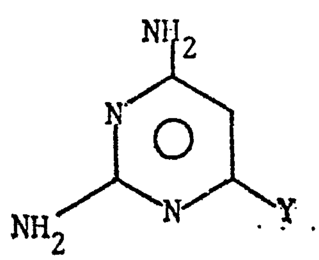

(XI)

wherein Y is a hydrogen or halogen atom or an alkylthio, aralkylthio or mercapto group, and then, in the case where Y is an alkylthio or aralkylthio group, removing this group by hydrogenolysis.

Suitably Z is a dialkylamino or cyclic amino group containing up to 10 carbon atoms, a dimethylamino group is particularly convenient. The reaction will be carried out in the presence of base under conditions well known to those skilled in the art of Mannich reactions. It has been found that the reaction may suitably be carried out in the presence of an alkoxide ion such as methoxide ion at an elevated temperature, suitably between 100 and 200$^{o}$C in a solvent having a suitably high boiling point, for example a glycol such as ethylene glycol. The dethiation is suitably carried out by hydrogenolysis in the presence of a transition metal catalyst; Raney nickel is particularly suitable for this purpose. This reaction will be carried out in a polar solvent, for example a $C_{1-4}$ alkanol such as methanol or ethanol; (iii) cleaving a group $R^{14}$, wherein $R^{14}$ is a protecting group, from a compound of the formula (XII):

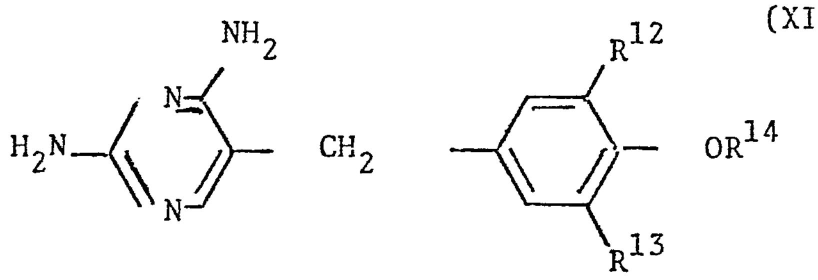

(XII)

wherein $R^{12}$, $R^{13}$ and $R^{14}$ are as hereinbefore defined. By the term "protecting group" is meant protecting groups conventionally used in the art to block free hydroxy groups, such as $C_{1-7}$ acyl groups, for example acetyl and benzoyl, acetal and ketal groups, such as tetrahydropyranyl, 2-methoxyethoxymethyl and methoxymethyl, and aralkyl groups, for example benzyl, phenylethyl and naphthylmethyl, and unconventional protecting groups, such as $C_{1-4}$ alkyl groups which may be cleaved from the compounds of the formula (XII). The reaction conditions will be those commonly used in removing protecting groups from hydroxy groups, i.e. mild acid hydrolysis or hydrogenolysis will be used for removing aralkyl groups, basic hydrolysis will be used for removing acyl groups and acid hydrolysis will be used for removing alkyl groups. Thus U.K. Patent No.1,261, 455 describes cleaving the benzyl group from 2,4-diamino-5-(p-benzyloxybenzyl)pyrimidine by hydrogenolysis and U.S. Patent No. 3,684,810 describes cleaving a methyl group from the 4-position of the

benzyl ring of 2,4-diamino-5-(3,4,5-trimethoxybenzyl) pyrimidine by acid hydrolysis; (iv) the reaction of a compound of the formula (XIII):

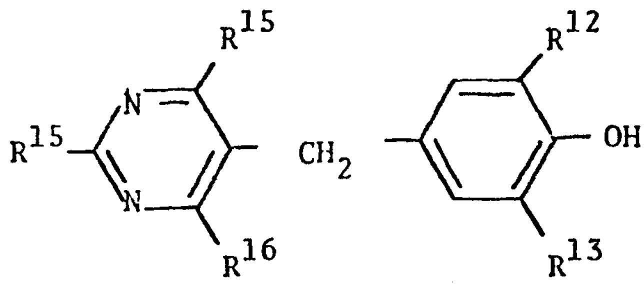

(XIII)

wherein $R^{15}$ is a leaving group or amino group and $R^{12}$ and $R^{13}$ are as hereinbefore defined, except that both the groups $R^{15}$ may not be amino groups, and $R^{16}$ is a hydrogen or halogen atom, with an aminating agent such as ammonia and thereafter, when $R^{16}$ is a halogen atom removing this by catalytic hydrogenolysis. Suitably $R^{15}$ is a halogen atom, such as a chlorine or bromine atom and $R^{16}$ is a chlorine or bromine atom. The aminating agent is suitably ammonia and the reaction will be carried out under the reaction conditions described in U.K. Patent Nos. 957797 and 1,132, 082. The reduction of $R^{16}$ when this is halogen will suitably be carried out by hydrogenolysis under the conditions described in Belgian Patent No.798724; (v) the reaction of guanidine as a guanidine salt

with a compound of the formula (XIV) or (XV):

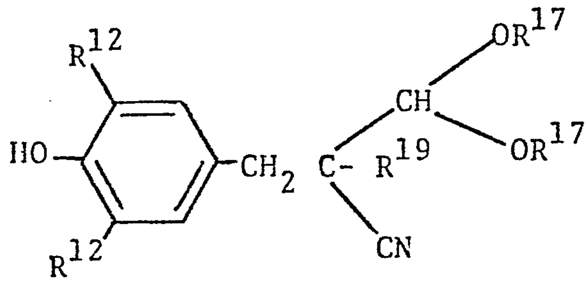

(XIV)

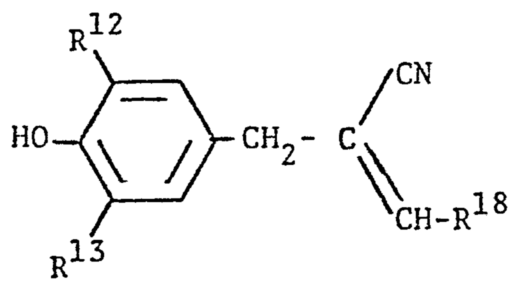

(XV)

or its corresponding benzal isomer; wherein $R^{17}$ is a lower alkyl group, $R^{18}$ is an amino or mono-or disubstituted amino group or an alkoxy group, $R^{19}$ is a formyl or alkoxycarbonyl group and $R^{12}$ and $R^{13}$ are as herein before defined. Suitably $R^{17}$ is a $C_{1-4}$ alkyl group and $R^{18}$ is a $C_{1-4}$ alkoxy group or an amino, $C_{1-4}$ alkylamino, benzylamino, di $C_{1-4}$ alkylamino, naphthylamino, optionally substituted anilino piperidino or morphilino group. Preferably $R^{18}$ is a methoxy, ethoxy, anilino or morpholino group. When $R^{19}$ is hydrogen, the guanidine will be in the form of a guanidine salt whilst when $R^{19}$ is formyl or

alkoxycarbonyl group it is unsalted.

The intermediates of the formulae (X), (XII), (XIV) and (XV) and certain intermediates of the formula (XIII) are novel and as such form an important further aspect of the present invention. The intermediates of the formula (XIII) are novel with the exception of those wherein $R^{12}$ and $R^{12}$ are both ethyl, n-propyl, iso-propyl or t-butyl.

The compounds of the formula (XII) may be prepared by any of the methods described in U.K. Patent No.1,375,162 and the compounds of the formulae (XIV) and (XV) may conveniently be prepared by the removal of a protecting group $R^{20}$ from the corresponding compounds of the formulae (XVI) and (XVII):

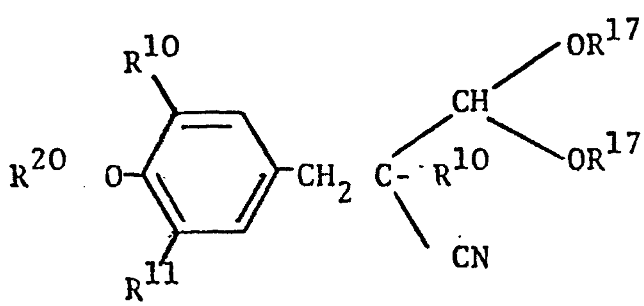

(XVI)

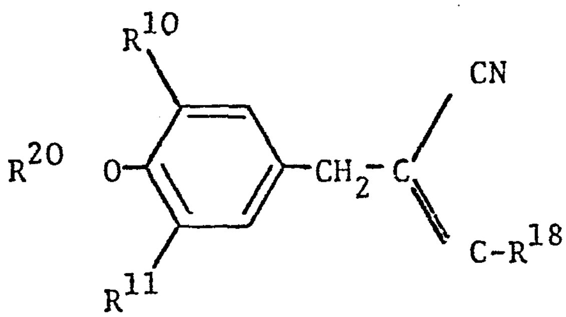

(XVII) and/or its corresponding benzal isomer

wherein $R^{10}$, $R^{11}$, $R^{17}$, $R^{18}$ and $R^{19}$ are as hereinbefore defined and $R^{20}$ is a protecting group. Suitable groups $R^{20}$ are those described in Protective Groups in Organic Chemistry Ed. J.F.W.McOmine, Plenum Press, London, 1973 and which are stable to alkaline conditions such as those removable by acid hydrolysis, such as acetal and ketal groups, and those removable by hydrogenolysis, such as aralkyl groups. Thus most suitably $R^{20}$ is a benzyl, naphthylmethyl or phenylethyl group or a tetrhydropyranyl, methoxymethyl or 2-methoxyethoxymethyl group and preferably $R^{20}$ is a benzyl or a methoxymethyl or 2-methoxyethoxymethyl group. The hydrogenolysis will suitably be carried out in a solvent such as an alkanol, for example methanol or ethanol, in the presence of a transition metal catalyst, palladium is particularly suitable, especially palladium on charcoal, at a temperature of between 0 and 80°C, and conveniently at room

temperature.

The compounds of the formula (XVI) and (XVII) may conveniently be prepared from a substituted benzaldehyde of the formula (XVIII):

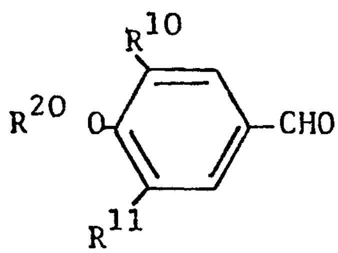

(XVIII)

wherein $R^{10}$, $R^{11}$ and $R^{20}$ are as hereinbefore defined, under the reaction conditions described for the preparation of structural analogues of compounds of the formula (XVI) and (XVII) described in U.K. Patent Nos. 957,797, 1,142,654, 1,133,766, 1,261, 455 and 1,375,162.

The known compounds included within the scope of formula (VI) may be prepared in an analogues manner to the novel compounds of the formula (VII).

The following examples are to illustrate the preparation of the compounds of the present invention and their pharmaceutical properties.

Antigonococcal Activity of 2,4-diamino-(3,5-di-alkyl-4-hydroxybenzyl)pyrimidines

The antigonococcal activity of 2,4-diamino-(3,5-diethyl-4-hydroxybenzyl)pyrimidine (compound 1), 2,4-diamino-(4-hydroxy-3,5-di-n-propylbenzyl)pyrimidine (compound 2), 2,4-diamino-(3-ethyl-4-hydroxy-5-n-propylbenzyl)pyrimidine (compound 3),24-diamino-(4-hydroxy-3,5-di-i-propylbenzyl)pyrimidine (compound 4) and 2,4-diamino-(3,5-di-t-butyl-4-hydroxybenzyl) pyrimidine (compound 5) and the antimeningococcal activity of compounds 2,3 and 4 were determined in mice by the following methods. The activities were measured against 17 strains of Neisseria gonorrhoeae and 5 strains of Neisseria meningitidis in Wellcotest Sensitivity Test Agar supplemented with 5% heated horse blood and 5% unheated lysed horse blood. In practice, 5% blood was added to 500ml quantities of the medium at 56$^{o}$C and, after being placed in a 80$^{o}$C water bath for 15 minutes and then cooled to 56$^{o}$C, the lysed blood and the test compounds were added. The medium was then immediately poured into plates. The test compounds were dissolved as the isothionates.

In the Screening Examination, which consisted of 6 separate tests, the minimum inhibitory concentrations (MIC's) were determined in one-third interval dilutions and then reexamined in one-half interval dilution.

The inoculum in the Screening Examination was from an 18-hour Mueller-Hinton heated-blood broth culture, and applied with a 1mm loop which was streaked along 1cm. Because the amount of growth produced by the strains varied, the inocula was not constant in these tests, in the Repeat Examination, the inocula were standardised to give "just confluent" growth. The MIC's were read as that concentration which produced 90% or more inhibition of growth as compared macroscopically with that of the control growth. The incubation period for the Screening Examination was 24 hours and for the Repeat Examination 48 hours.

As standards, *Escherichia coli* (CN 314 ) and trimethoprim (TMP) were included in each test. The results are shown in Table 1.

Using the above method the activities of compound 5 was measured against the same 17 strains of

Neisseria gonorrhoeae and 5 strains of Neisseria meningitidis in Difco G.C. Agar.

Also shown in Table 1 is the concentration of each of the compounds in the blood urine and tissues of mice after oral administration. These concent rations were determined as described hereafter. The compounds, suspended in 0.1% methylcellulose, were administered by stomach tube to groups of 3 mice, the dose was 2.0mg per 20g mouse and blood was collected from the orbital sinus at 1, 2 and 4 hours after the dosing. Urine was collected directly from each animal at the 4th hour, after which the animals were killed. Liver, lung, brain and muscle was taken from each animal and homogenised in an equal volume (w/v) of water in a Polytron Homogenizer. The suspensions were taken to be 1:2 extracts of the tissues.

The concentrations of the drugs were determined microbiologically, using Bacillus pumilus, those in the urine determined both before and after 18 hours incubation with Glusalase (gluecuronidase and sulfatase).

**TABLE 1** Antigonococcal Activity* and Blood, Urine and Tissue Concentrations of 2,4-Diamino-(3,5-di-alkyl-4-hydroxybenzyl)pyrimidines

| | Antigonococcal Activity | | | | | Pharmokinetics 2mg/mouse P.O. | | | | | | | | | |
|---|---|---|---|---|---|---|---|---|---|---|---|---|---|---|---|
| | v.N.gonorrhacae | | | v.N.meningitidis | v.E.coli | Conc. in blood µg/ml at | | | Concentration µg/ml at 4HRS in: | | | | | | |
| | W.S.T.A. | | G.C.A. | | (CN314) | 1HR | 2HR | 4HR | Brain | Lung | Liver | Muscle | Urine Direct | After glucolase |
| TMP | 1 | 1 | 1 | 1 | 1 | 2.15 | 0.28 | <0.02 | <0.04 | | 1.08 | <0.04 | 200 (50%) | 400 |
| COMPOUND 1 | 7 | - | - | - | - | 1.11 | 0.41 | 0.40 | 0.34 | 1.75 | 1.94 | <0.03 | 56 (7%) | 790 |
| COMPOUND 2 | 21 | 17** | - | 11 | 0.03 | 1.36 | 0.50 | 0.44 | 0.5 | 1.8 | 3.75 | <0.03 | 16 (2.5%) | 650 |
| COMPOUND 3 | 21 | 18** | - | 7 | >0.1 | 0.88 | 0.9 | 0.8 | 0.44 | 3.6 | 5.0 | 0.21 | 39 (2.2%) | 1800 |
| COMPOUND 4 | 18 | 17** | 13 | 7 | 0.03 | 1.38 | 1.08 | 0.65 | 0.46 | 2.6 | 8.3 | <0.03 | 95 (50%) | 190 |
| COMPOUND 5 | - | - | 7 | - | - | 4.25 | 4.0 | 3.0 | 4.4 | 11.2 | 6.0 | <0.26 | 100 (42%) | 240 |

W.S.T.A. = Wellcotest Sensitivity Test Agar

G.C.A. = Difco G.C. Agar

* expressed as activity versus TMP activity

** Repeat Examination

0006987

EXAMPLE 1

2,4-Diamino-5-(3,5-di-t-butyl-4-hydroxybenzyl) pyrimidine

2,4-Diamino-5-hydroxymethylpyrimidine (7.0g, 0.05mol), 2,6-di-t-butylphenol (10.3g, 0.05mol), 225 ml of glacial acetic acid, and 6.7 ml of concentrated hydrochloric acid were heated together under reflux for 11 hours. The solvent was removed, and the yellow oil was taken up in 0.5M sodium bicarbonate solution and chloroform. The aqueous layer was adjusted to pH 8.5 and extracted several times with chloroform. The combined chloroform extracts were dried (magnesium sulphate) and evaporated to dryness. The resultant residue was passed down a silica gel column (eluant chloroform/ methanol in a 30:1 ratio initially and then in a 18:1 and a 9:1 ratio) to give two products, the second product off the column being identified by n.m.r. as the title compound, m.p. 208.5-210.5$^{o}$ (ex methanol) (10.3% yield).

EXAMPLE 2

2,4-Diamino-5-(3,5-di-t-butyl-4-hydroxybenzyl) pyrimidine

7.1g (0.05mol) of 5-hydroxymethyluracil (R.E. Cline, et al. J. Am. Chem. Soc. 81, 2521 (1959)) 10.3g (0.05mol) of 2,6-di-t-butylphenol, 200 ml of glacial acetic acid, and 7 ml of concentrated hydrochloric acid were heated together at reflux for 6 hours. The solvent was removed, yielding a white solid. One hundred ml of water was added and the pH adjusted to 6.8. The heavy precipitate was filtered, then heated in 250 ml of absolute ethanol, and 3.7 g of white precipitate (crop A) filtered. The filtrate was diluted with 250 ml of water, and then chilled. 2.63 g of light yellow precipitate (crop B) was filtered. Both precipitates were the desired product, 5-(3,5-di-t-butyl-4-hydroxybenzyl)uracil, as shown by TLC and NMR. The yield was 38.4%.

3.3 g of crop A was heated in 40 ml of phosphorus oxychloride. In ½ hour the compound dissolved, and it was heated 2 hours longer. The solvent was removed leaving a yellow oil which was slurried in 100 ml of ice water. The pH was adjusted to 6.8 with sodium carbonate. The precipitate which formed was filtered

giving 2.60 g of a white precipitate. The yield was 70.9% crude. NMR and TLC showed traces of impurities but the major compound was 2,4-dichloro-5-(3,5-di-t-butyl-4-hydroxybenzyl)pyrimidine.

6.6g of crop B (from two preparations) was heated in 60 ml of phosphorus oxychloride and worked up as above. The precipitate which was filtered was oily and it was dissolved in chloroform and extracted twice with 50 ml portions of water (pH 5). The chloroform was evaporated, and the material was recrystallized from absolute EtOH giving 2.21 g of the dichloro compound in two crops, 30.2% yield, m.p. 145.5-146.5°. The mother liquor contained 2.63 g and showed two spots on TLC.

1.0 g (2.7mmol) of 2,4-dichloro-5-(3,5-di-t-butyl-4-hydroxybenzyl)pyrimidine from the above reaction was aminated using 35 ml of saturated ammonia in absolute EtOH in a bomb at 140° for 7 hours. The solvent was evaporated, and the residue was taken up in chloroform/methanol (3:1) and 50 ml of 0.5M $NaHCO_3$ solution (adjusted to pH 8.5). The chloroform/methanol layer was evaporated and put on a silica gel column using chloroform/methanol (30:1, later 18:1) elution. There was recovered 0.15g of the desired

compound, 2,4-diamino-5-(3,5-di-t-butyl-4-hydroxybenzyl)pyrimidine (17%). Other products were also isolated. NMR and MS confirmed the structures of the product obtained.

EXAMPLE 3

2,4-Diamino-5-(3,5-di-t-butyl-4-hydroxybenzyl) pyrimidine

103 g (0.5mol) of 2,6-di-t-butylphenol was dissolved in 500 ml of EtOH. 310 g of 20% dimethylamine in absolute EtOH was added with stirring at room temperature. Then the solution was chilled to 5$^o$ and 75 g of 37% formaldehyde (0.93 mole) was added. Then the solution was allowed to come to room temperature and stirred overnight. Then it was refluxed for 3 hours, cooled, and poured into 2 l. of ice water. On standing, a precipitate formed, which was filtered, and recrystallized from hexane giving a 90% yield of 2,6-di-t-butyl-4-N,N-dimethylaminomethylphenol.

5.6 g (0.05mol) of uracil and 13.15 g (0.05mol) of 2,6-di-t-butyl-4-N,N-dimethylaminomethylphenol was dissolved in 65 ml. of ethylene glycol. Then 2.7g (0.05mol) of sodium methylate was added. The reaction was heated for 6 hours with nitrogen blowing

through the reaction, and the dimethylamine released was collected and neutralized in an equivalent amount of aqueous $H_2SO_4$ solution. After cooling, 250 ml of water was added, followed by 5.7 ml of glacial acetic acid to pH 4.5. The precipitate was filtered and washed with water and ether, giving 12.98g of 5-(3,5-di-t-butyl-4-hydroxybenzyl)uracil (78.7%). TLC and NMR showed that the compound was correct.

11.6g (0.03mol) of above compound was chlorinated using 130 ml of phosphorus oxychloride. The reaction was heated for 3½ hours and allowed to cool. Then the solvent was removed, and 300 ml of ice water was added. Sodium carbonate was added until the pH was 7.2, and the precipitate was filtered giving 8.04 g, 62.5% yield, of 2,4-dichloro-5-(3,5-di-t-butyl-4-hydroxybenzyl) pyrimidine. NMR confirmed the structure, and TLC indicated slight impurities.

Amination of the above material was done in two separate bomb reactions. The first one used 4.0g (0.011 mol) of the above material and 50 ml of saturated ammonia/ethanol in a bomb at $140^o$ for hours. The solvent was removed, and 100 ml of 0.05M $NaHCO_3$ solution was added, and the pH adjusted to 8.5. The aqueous layer was extracted with four times 100 ml

of chloroform, and the chloroform layer was evaporated.

The second amination used 3.77 g(0.010 mol) of the dichloropyrimidine, absolute ethanol, and 30 ml of saturated ammonia/ethanol solution. The extra ethanol was added to dissolve the compound initially before the reaction started, but the compound was not very soluble in the ethanol. The bomb was heated at 165$^{o}$ for 8 hours. It was worked up as in Experiment 2, and the two chloroform soluble fractions were purified on a silica gel column using chloroform elution, followed by chloroform/methanol, 50:1 and then 25:1 ratio. The fractions corresponding to 2,4-diamino-5-(3-,5-di-t-butyl-4-hydroxybenzyl) pyrimidine were combined giving 1.72g, 24.7% crude yield, and then recrystallized from methanol to give 1.25g of product. CHN, TLC and NMR confirmed the product. CHN: Calc.: C-69.48,H-8.59, N-17.06; Found: C-69.39, H-8.69, N-17.06. M.P. 208-210$^{o}$C. Other products were also isolated from the column. Increasing the temperature of the bomb reaction increased the amount of desired diaminated product.

EXAMPLE 8

Tablet Formation

| | |
|---|---|
| 2,4-Diamino-5-(3,5-di-t-butyl-4-hydroxybenzyl)pyrimidine | 100.0 mg |
| Lactose | 85.0 mg |
| Potato starch, dried | 14.3 mg |
| Magnesium stearate | 0.7 mg |
| | 200.0 mg |

The pyrimidine, lactose and potato starch were blended together and granulated with water. The granules were dried, mixed with magnesium stearate and compressed in the normal manner to give tablets of satisfactory properties and appearance.

Acute oral toxicity of 2,4-diamino -5-(3,5-diisopropyl-4-hydroxybenzyl) pyrimidine lactate salt $LD_{50}$ (rats) greater than 1000mg/kg $LD_{50}$ (mice) female 2890mg/kg; male 2830mg/kg.

What we claim is

1). A pharmaceutical composition which comprises a compound of the formula (VI):

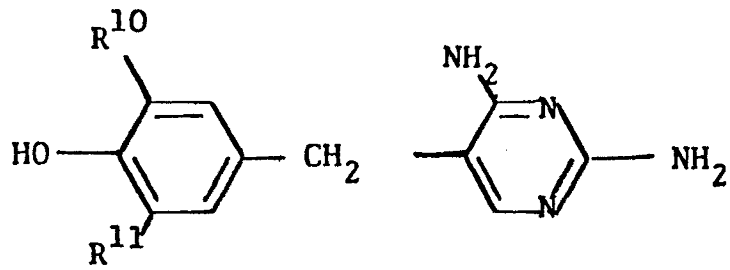

(VI)

wherein $R^{10}$ and $R^{11}$, which may be the same or different, are $C_{2-4}$ alkyl groups, or a pharmaceutically acceptable salt thereof, together with a pharmaceutically acceptable carrier.

2). A pharmaceutical composition according to claim 1 wherein $R^{10}$ and $R^{11}$ are i-propyl, s-butyl, i-butyl or t-butyl.

3). A pharmaceutical composition which comprises 2,4-diamino-(4-hydroxyl-3,5-di-i-propylbensyl) pyrimidine, or a pharmaceutically acceptable salt thereof, together with a pharmaceutically acceptable carrier.

4). A pharmaceutical composition which comprises a compound of the formula (VI) together with a sulforamide as defined in any one of claims 1-3, or a pharmaceutically acceptable salt thereof,

5). A compound of the formula (VI), or a pharmaceutically acceptable salt thereof, as defined in claim 1, for use in medicine for the treatment of microbial infections.

6). A compound of the formula (VI), or a pharmaceutically acceptable salt thereof, as defined in claim 1, for use for the treatment of bacterial infections.

7). A compound of the formula (VI), or a pharmaceutically acceptable salt thereof, as defined in claim 1, for use in the treatment of anaerobic infections.

8). 2,4-Diamino-(4-hydroxy -3,5-di-i-propylbenzyl) pyrimidine for use in medicine for the treatment of microbial infections.

9). A compound of the formula (VII):

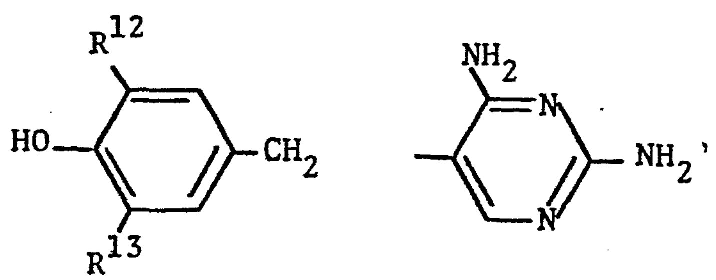

(VII)

or a pharmaceutically acceptable salt thereof, wherein $R^{12}$ and $R^{13}$, which may be the same or different are propyl or butyl, except that $R^{12}$ cannot be i-propyl when $R^{13}$ is i-propyl

U.K. Patent No.1,128,234 claims inter alia compounds of the formula (II):

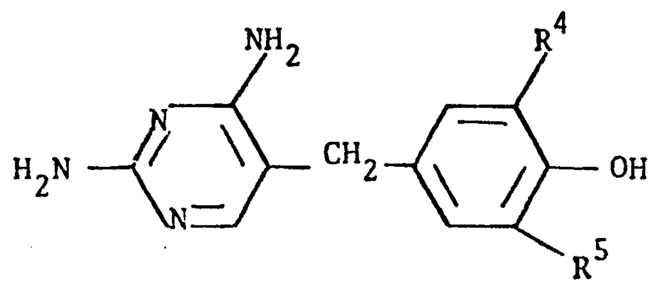

(II)

wherein $R^4$ and $R^5$, which may be the same or different are hydrogen, halogen, $C_{1-4}$ alkyl or $C_{1-4}$ alkoxy. However, only those compounds of the formula (II) wherein $R^4$ and $R^5$ are methoxy are exemplified, there being no exemplification of those compounds wherein $R^4$ and $R^5$ are $C_{1-4}$ alkyl groups.

Belgian Patent No. 846,937 claims compounds of the formula (III):

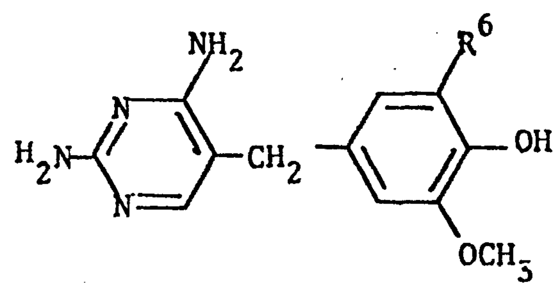

(III)

wherein $R^6$ is a $C_{1-4}$ alkyl group or a halogen atom. No mention is made of these compounds being active

against <u>Neisseria</u>.

A further group of 2,4-diamino-5-benzylpyrimidines found to have antibacterial activity are described in U.K. Patent No. 1,374,162 and have the formula (IV):

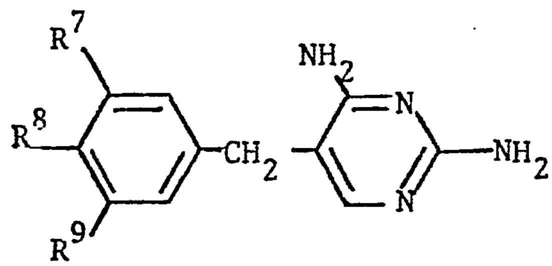

(IV)

wherein $R^7$ and $R^9$ are the same or different $C_{2-4}$ alkyl groups and $R^8$ is a $C_{1-12}$ alkyl or $C_{1-12}$ alkoxy group. The compounds wherein $R^8$ is an alkoxy group may be prepared (U.K. Patent Nos. 1375162 and 1413471) by the alkylation of a compound of the formula (V):

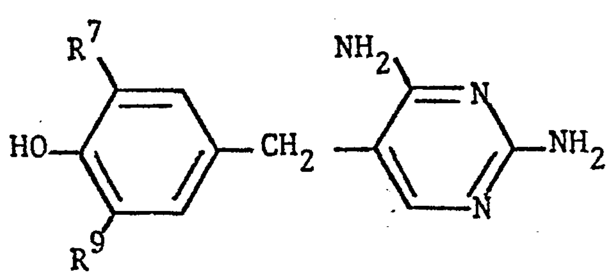

(V)

wherein $R^7$ and $R^9$ are as hereinbefore defined. Although the compounds of the formula (V) were described in U.K. Patent No. 1,413,471 as being useful

wherein $R^{15}$ is a leaving group or amino group and $R^{12}$ and $R^{13}$ are as hereinbefore defined, except that both the groups $R^{15}$ may not be amino groups, and $R^{16}$ is a hydrogen or halogen atom, with an aminating agent and thereafter, when $R^{16}$ is a halogen atom, removing this by catalytic hydrogenolysis.

(v) the reaction of guanidine as a guanidine salt with a compound ofthe formula (XIV) or (XV):

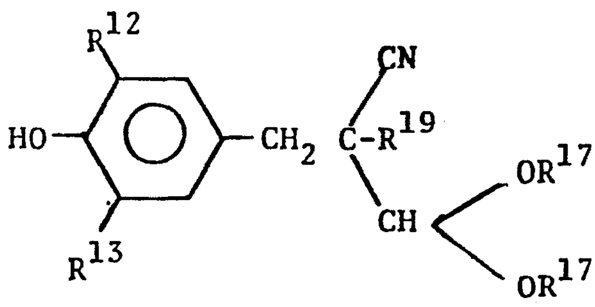

(XIV)

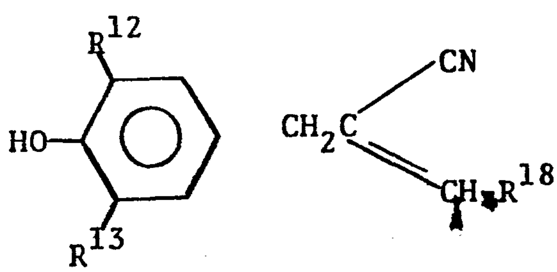

(XV)

or its corresponding banzal isomer;

wherein $R^{17}$ is a lower alkyl group, $R^{18}$ is an amino or mono or disubstituted amino group or an alkoxy group, $R^{19}$ is a hydrogen atom or a formyl or alkoxycarbonyl group, and $R^{12}$ and $R^{13}$ are as hereinbefore defined.

0006987

European Patent Office

# EUROPEAN SEARCH REPORT

Application number
EP 79101596.9

## DOCUMENTS CONSIDERED TO BE RELEVANT

| Category | Citation of document with indication, where appropriate, of relevant passages | Relevant to claim |
|---|---|---|
| D | GB-A-875,562 (Wellcome)<br>* Totality* | 1-10 |
| D | GB-A-1 128 234 (Wellcome)<br>* Totality* | 1-10 |
| | GB-A-1 176 395 (Hoffmann)<br>* Totality* | 1-4 |
| D | GB-A-1 375 162 (Wellcome)<br>* Examples 5 to 8, 11* | 1-10 |
| D | GB-A-1 413 471 (Wellcome)<br>* Examples 1,2,3* | 5-10 |
| | GB-A-1 413 472 (Wellcome)<br>* Examples 1,2,3* | 5-10 |
| A | US-A-3 684 810 (Hoffmann)<br>* Totality* | 10 |
| A | DE-A-2 546 667 (Heumann)<br>* Totality* | 1-10 |

CLASSIFICATION OF THE APPLICATION (Int. Cl.³)

C07D239/48
A61K31/505

TECHNICAL FIELDS SEARCHED (Int.Cl.³)

C07D239/48
A61K31/505

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| WIEN | 27. Juli 1979 | LUX |

EPO Form 1503.1 06.78